# EUROPEAN PATENT APPLICATION

(11) **EP 4 389 134 A1**
(43) Date of publication of application: **26.06.2024**
(21) Application number: 22306952.7
(22) Date of filing: 20.12.2022
(51) Int. Cl.: A61K 35/741, A61P 31/04

(54) **FAECALIBACTERIUM PRAUSNITZII STRAIN CNCM I-4573 FOR THE TREATMENT AND PREVENTION OF CLOSTRIDIOIDES DIFFICILE INFECTION**

(71) Applicant: Exeliom Biosciences, 21000 Dijon (FR); Institut national de recherche pour l'agriculture, l'alimentation et l'environnement (INRAE), 75007 Paris (FR)
(72) Inventor: LANGELLA, Philippe, 78140 VELIZY (FR); PECHINE, Séverine, 91190 GIF SUR YVETTE (FR); CAMPIDELLI, Camille, 78690 SAINT REMY L'HONORE (FR); CHATEL, Jean-Marc, 92190 MEUDON (FR); SOKOL, Harry, 75015 PARIS (FR); JANOIR, Claire, 92190 MEUDON (FR); HADIDA, Benjamin, 75011 PARIS (FR); RUFFIE, Pauline, 33400 TALENCE (FR)
(74) Representative: Cabinet Nony

(57) **Abstract**

The present invention relates to a bacterial strain of the species *Faecalibacterium prausnitzii* deposited with the CNCM under accession number CNCM I-4573, and/or a culture supernatant thereof, for use in the treatment and/or prevention of a *Clostridioides difficile* infection in an individual.

## Description

### Field of the invention

The present invention relates to a *Faecalibacterium prausnitzii* strain for use thereof in the treatment and prevention of a *Clostridioides difficile* infection in an individual.

### Prior art

*Clostridioides difficile* (also referred to as *C. difficile,* and formerly known as *Clostridium difficile*) is an anaerobic, spore-forming, toxin- producing, gram-positive bacterium that is transmitted among humans via the fecal-oral route. Elderly, immunocompromised individuals whose gut microbiota have been disrupted by antibiotic therapy have the greatest risk of contracting this highly-contagious, life-threatening, and potentially-fatal diarrheal disease. After highly-virulent strains began appearing during the early 2000s, *C*. *difficile* emerged as a major, globally-distributed enteric pathogen.

Generally, *C*. *difficile* is regarded as a nosocomial pathogen, being hospital-acquired, even though *C*. *difficile* infections can also occur also outside hospitals.

However, the disease is now appearing more frequently in individuals once considered to be at low risk. These cases tend to occur in younger individuals. Recent antibiotic exposure is an important risk factor in this population, but use of gastric acid suppressants and co-morbidities such as inflammatory bowel disease, chronic kidney disease, immunodeficiency disease, malignant lesions and solid organ transplants have also been associated with *C*. *difficile* (CDI). In some rare cases, the infection can even spontaneously appear in otherwise healthy individuals in the absence of antibiotic exposure.

*C*. *difficile* is responsible for 15-25% of cases of antibiotic-associated bacterial diarrhoea and is considered the leading cause of healthcare-associated diarrhoea in adults. After a first episode, the risk of first recurrence is high and problematic (about 20%) and it increases further after a first recurrence reaching up to 60%. The symptoms associated with this infection can range from simple diarrhoea to pseudomembranous colitis.

*C*. *difficile* is accordingly classified as one of the top three urgent antibiotic resistance threats by the Centers for Disease Control and Prevention (CDC), and CDI has become the most common cause of antibiotic-associated diarrhea and gastroenteritis-associated death in developed countries.

Currently, the standard treatment for CDI is antibiotics, with vancomycin or fidaxomicin being the most commonly prescribed compounds. Metronidazole is only used if vancomycin or fidaxomicin are not available. Fecal microbiota transplant (FMT) has only been used as a last resort following multiple recurrences. Probiotics have also been used as a preventive treatment with mixed results while vaccines are not yet available. Within healthcare facilities, preventive measures include judicious use of antibiotics and infection control practices. Use of monoclonal antibodies has also been studied with the aim of improving host resistance to CDI, however this approach has yielded mixed results.

There is a need for novel substances, in particular probiotics, or compositions comprising them, for the treatment and/or prevention of an infection by *C*. *difficile.*

There is in particular a need for novel substances, in particular probiotics, or compositions comprising them, able to decrease the risk of *C*. *difficile* infection following a gut microbiota perturbation with antibiotics and/or Proton pump inhibitors (PPI).

There is in particular a need for novel substances, in particular probiotics, or compositions comprising them, able to decrease the risk of *C*. *difficile* infection recurrence after a given episode.

There is in particular a need for novel substances, in particular probiotics, or compositions comprising them, able to delay intestinal colonisation by *C*. *difficile.*

There is also a need for novel substances, in particular probiotics, or compositions comprising them, allowing reduced, or even the absence of, symptoms following infection by *C*. *difficile.*

There is also a need for novel substances, in particular probiotics, or compositions comprising them, allowing limiting intestinal colonization by the bacteria, a faster elimination of *C*. *difficile* from the intestine and advantageously then a reduced or even the absence of symptoms following infection.

### Summary of the invention

The objective of the present invention is to provide novel substances, in particular probiotics, and compositions for the treatment and/or prevention of a *Clostridioides difficile* infection in an individual.

In the context of the present invention, the terms *"prevent", "prevention"* and *"preventing"* denote the reduction to a lesser degree of the risk or of the probability of occurrence of a given phenomenon, that is to say, in the present invention, a *C*. *difficile* infection, and in particular the development of symptoms following infection by *C*. *difficile.* In particular, the terms *"prevent", "prevention"* and *"preventing"* denote the reduction to a lesser degree of the risk or of the probability of a first episode of *C*. *difficile* infection (CDI) and/or the reduction to a lesser degree of the risk or of the probability of recurrence of *C*. *difficile* infection in an individual. For the prevention of the first episode, the target population may in particular correspond to any patient for whom an antibiotic treatment and/or a treatment comprising administration of Proton pump inhibitors (PPI) is prescribed, and in particular any patient for whom an antibiotic treatment is prescribed.

Recurrence of CDI is defined as recurrence of diarrhea with a positive test for *C. difficile* toxin within 8 weeks of completion of effective anti-*C*. *difficile* therapy.

As used herein, the terms *"treating", "treat"* or *"treatment"* include alleviation of the symptoms associated with a specific disorder or condition and/or elimination of said symptoms as well as the complete disappearance of the considered disorder or condition.

The present invention is based on the discovery, by the present inventors, of the ability of the *Faecalibacterium prausnitzii* strain deposited with the CNCM under accession number CNCM 1-4573 to prevent and/or treat a *Clostridioides difficile* infection in an individual.

According to the experimental results of the inventors, a specific strain of *Faecalibacterium prausnitzii* (*F. prausnitzii*) deposited with the CNCM on January 21, 2012, under accession number CNCM I-4573 by Institut National de la Recherche Agronomique (INRA) has the unexpected capacity to treat and prevent, *in vivo,* the infection of an individual by *C*. *difficile,* and in particular the unexpected capacity to delay intestinal colonisation by *C*. *difficile* of said individual, to rapidly eliminate *C*. *difficile* from the intestine, and to reduce symptoms following infection by *C*. *difficile* or even to prevent the appearance of any symptom following infection by *C*. *difficile.*

According to a first subject, the present invention relates to a bacterial strain of the species *Faecalibacterium prausnitzii* deposited with the CNCM under accession number CNCM I-4573, and/or a culture supernatant thereof, for use in the treatment and/or prevention of a *Clostridioides difficile* infection in an individual.

This strain identified by the inventors is therefore a probiotic strain which may be used for the applications indicated herein.

An individual according to the invention may be selected from the group consisting of human beings; *Suidae,* in particular pigs, more particularly piglets; *Bovinae,* in particular cattle, more particularly bulls, and more particularly steers; *Canidae,* in particular dogs; *Equidae,* in particular horses; and *Phasianidae,* in particular poultry, more particularly chickens; preferably a mammal, and more particularly a human being (J. Scott Weese; Journal of Veterinary Diagnostic Investigation 2020, Vol. 32(2) 213-221).

The bacterial strain of the species *Faecalibacterium prausnitzii* deposited with the CNCM under accession number CNCM I-4573 may be administered to the said individual in a live, inactive and/or dead form, preferably in a live or inactive form, more particularly in a live form.

A live bacterial form means a bacterial strain which is cultivable and/or metabolically active, in particular cultivable and metabolically active.

An inactive form of a bacterial strain according to the invention may for example be a lyophilisate, prepared using method of the art well known by the skilled artisan.

According to an embodiment, the *Clostridioides difficile* infection may occur after the use of at least one antibiotic medication and/or at least one Proton pump inhibitors (PPI) medication by the individual. The bacterial strain of the species *Faecalibacterium prausnitzii* deposited with the CNCM under accession number CNCM I-4573, and/or the culture supernatant thereof, may be administered simultaneously and/or separately, in particular simultaneously, with the at least one antibiotic medication and/or at least one PPI medication. The bacterial strain of the species *Faecalibacterium prausnitzii* deposited with the CNCM under accession number CNCM I-4573, and/or the culture supernatant thereof, may be administered simultaneously with and/or after the at least one antibiotic medication and/or the at least one PPI medication.

The bacterial strain for use thereof, and/or the culture supernatant thereof, according to the invention may be included in a composition comprising a physiologically acceptable medium, preferably in an oral or rectal composition, and more particularly preferably in a pharmaceutical product.

The term *"physiologically acceptable medium"* is intended to denote a medium which is compatible with the body of the individual to whom said composition must be administered. It is, for example, a non-toxic solvent such as water. In particular, said medium is compatible with oral administration.

A composition of the invention is preferably for oral or rectal administration, in particular for oral administration.

A composition of the invention for oral administration may be chosen from the group consisting of a food product, a beverage, a pharmaceutical product, a nutraceutical, a food additive, a food supplement and a milk product and is, in particular, in the form of a pharmaceutical product.

A composition for use according to the invention may be suitable for the administration of a daily dose representing from 10⁴ to 10¹⁵ total cell count (TCC), more preferably from 10⁸ to 10¹³ TCC of the *F*. *prausnitzii* strain of the invention as a medicament, for example as a daily dose representing 10¹³ TCC of the bacterial strain according to the invention.

### Figures' legends

Figure 1 illustrates the evaluation of weight loss in mice treated or not with *F*. *prausnitzii* I-4573 according to the invention. Mice were weighed to monitor the development of clinical signs after infection with *C*. *difficile.* Two groups of mice are tested: mice treated with PBS- Glycerol 16% (vehicle); mice treated with *F. prausnitzii* strain I-4573.
   Abscissa: Days (from D0 to D20, D0 being the day the mice, previously treated with antibiotics, are fed with *C*. *difficile* R20291). Ordinate: Weight of the mice (% compared to their weight at D0).
Figure 2 illustrates survival of mice treated with PBS- Glycerol 16% (vehicle) or treated with *F. prausnitzii* strain I-4573 of 7 days
   Abscissa: Days (from D0 to D7). Ordinate: Probability of survival (between 0 and 1).
Figure 3 illustrates the colonization of mice (10 mice per group) by *C*. *difficile* R20291 over time (from D0 to D20 - D0 being the day the mice, previously treated with antibiotics, are fed with *C*. *difficile* R20291).

Abscissa (arrow), from left to right: D0, D2, D3, D4, D5, D6, D8, D10, D14 and D20. Ordinate: % of mice with detectable *C*. *difficile* in feces.

### Detailed description of the invention

The present inventors have carried out thorough studies in order to identify the capacity of a specific strain of *Faecalibacterium prausnitzii* to treat and/or prevent *Clostridioides difficile* (*C. difficile*) infection in an individual.

Indeed, the inventors have determined, unexpectedly, that the *F*. *prausnitzii* strain I-4573 has the capacity to not only delay intestinal colonisation by *C*. *difficile,* but to also rapidly eliminate *C*. *difficile* from the intestine, and to significantly reduce symptoms following infection by *C*. *difficile,* in particular to prevent the appearance of any symptom following infection by *C*. *difficile,* in an individual.

### Faecalibacterium prausnitzii strain of the invention

*F. prausnitzii* is a major member of the phylum *Firmicutes* and is part of the commensal bacteria that are the most abundant in the microbiota of the healthy human large intestine.

*F. prausnitzii* is an extremely oxygen-sensitive (EOS) bacterium which is therefore difficult to culture, even under anaerobic conditions (Duncan et al. 2002, Int. J. Syst. Evol. Microbiol. 52(Pt 6): 2141-6 and Lopez-Siles et al. Appl. Environ Microbiol. January 2012; 78 (2):420-8). *F. prausnitzii* is in particular known to be one of the most abundant butyrate-producing bacteria in the human digestive tract, the butyrate short-chain fatty acid being very important in intestinal physiology, systemic functions and beneficial effects on human health (Macfarlane and Macfarlane (2011), J. Clin. Gastroenterol. 45 Suppl: S120-7).

A suitable daily dose of a bacterial strain according to the invention may be from 10⁷ to 10¹⁴ colony-forming units (cfu) of the *F*. *prausnitzii* strain of the invention as a medicament.

Daily dosages may also range from 10⁴ to 10¹⁵ total cell count (TCC), more preferably from 10⁸ to 10¹³ TCC of the *F*. *prausnitzii* strain of the invention as a medicament, for example as a daily dose equivalent to 10¹³ TCC.

The *F*. *prausnitzii* strain specifically implemented in the present invention is the bacterial strain of the species *Faecalibacterium prausnitzii* deposited with the CNCM under accession number CNCM I-4573.

As previously mentioned, a *Faecalibacterium prausnitzii* strain of the invention, and/or a culture supernatant thereof, is for use in the treatment and/or prevention of a *Clostridioides difficile* infection in an individual.

A *Faecalibacterium prausnitzii* bacterial strain deposited to the CNCM under the accession number I-4573 for use according to the invention may be in an isolated form. By "*isolated form*" according to the invention, it is meant in a form isolated from any fecal material, or intestinal sampling. Accordingly, a bacterial strain of the invention is preferably not administered to a patient in the form of a Fecal Microbiota Transplant (FMT). Methods for isolating a bacteria from the gut microbiota are well known to the man skilled in the art, such as for example illustrated in Foditsch et al. (PLoS One. 2014; 9(12): e116465).

An individual according to the invention may be selected from the group consisting of human beings; *Suidae*, in particular pigs, more particularly piglets; *Bovinae*, in particular cattle, more particularly bulls, and more particularly steers; *Canidae*, in particular dogs; *Equidae*, in particular horses; and *Phasianidae*, in particular poultry, more particularly chickens. In particular, an individual according to the invention is a mammal, in particular a human being.

The strain of the invention is a probiotic, the activity of which is located in the intestines. A probiotic bacterium according to the invention denotes a bacterium which, when it is ingested live in sufficient amounts, may exert beneficial effects on an individual's health.

Consequently, the bacterial strain of the species *Faecalibacterium prausnitzii* deposited with the CNCM under accession number CNCM I-4573 may be administered to an individual in need thereof in a live, inactive and/or dead form, and is preferably administered in a live form in the intestines.

The bacterial strain of the invention may be administered to the digestive tract of an individual to be treated in various ways, namely orally or rectally. A bacterium according to the invention is preferably administered orally.

According to one preferred embodiment, the bacterial strain of the invention is included in a composition comprising a physiologically acceptable medium. Such a composition is preferably for oral or rectal, in particular oral, administration, and in particular in the form of a pharmaceutical product.

### Compositions

The present invention also relates to a composition for use according to the invention, the composition comprising, in a physiologically acceptable medium, at least the *Faecalibacterium prausnitzii* bacterial strain I-4573 of the invention.

A composition for use according to the invention is intended for the digestive tract, in particular the intestines.

Consequently, a composition for use according to the invention may be chosen from an oral or rectal composition. A composition of the invention is preferably an oral or rectal composition, more preferably an oral composition.

A composition for use of the invention being an oral composition is intended for oral administration to an individual.

A composition for use of the invention being a rectal composition is intended for rectal administration to an individual.

An oral composition may be in the form of a suspension, a tablet, a pill, a capsule, a granule or a powder.

A composition for use according to the invention for oral administration may be chosen from the group consisting of a food product, a beverage, a pharmaceutical product, a nutraceutical, a food additive, a food supplement or a milk product and is, in particular, a pharmaceutical product.

According to one preferred embodiment, a composition for use according to the invention is a pharmaceutical product.

A pharmaceutical product for oral administration may be present in capsules, gel capsules, soft capsules, tablets, sugar-coated tablets, pills, pastes, lozenges, gums, oral solutions or emulsions, a syrup or a gel.

For example, a pharmaceutical product according to the invention for oral administration may be in a capsule, in particular in a capsule under the form of a lyophilisate, preferably together with pharmaceutically acceptable excipients.

Advantageously, a composition for use according to the invention, intended for oral administration, may be provided with a gastricjuice-resistant coating, in order to ensure that the bacterial strain of the invention included in said composition can pass through the damaged stomach. The release of the bacterial strain may thus take place for the first time in the upper intestinal tract.

A pharmaceutical product for oral use according to the invention may also comprise a sweetener, a stabilizer, an antioxidant, an additive, a flavoring agent and/or a dye.

The formulation thereof is carried out by means of the usual methods for producing sugar-coated tablets, gel capsules, gels, controlled-release hydrogels, emulsions, tablets or capsules.

A composition for use containing the bacterial strain of the invention may be administered intrarectally.

A rectal administration may in particular be carried out in the form of a suppository, an enema or a foam, in particular an enema.

A composition for use according to the invention may also be in the form of a nutritional composition.

A nutritional composition for use according to the invention may be in the form of a yogurt, a cereal bar, breakfast cereals, a dessert, a frozen food, a soup, a pet food, a liquid suspension, a powder, a tablet, a gum or a candy.

In particular, a composition for use of the invention may be suitable for the administration of a daily dose representing from 10⁷ to 10¹⁴ colony-forming units (cfu) of the strain according to the invention as a medicament, preferably a daily dose equivalent to 10¹¹ cfu.

For example, a composition for use according to the invention may be administered to an individual requiring it, at a single daily dose of 1 g containing the I-4573 bacterial strain of the invention in an amount equivalent to a dose of between 10⁷ and 10¹⁴ cfu, preferably 10¹¹ cfu.

In another example, a composition for use according to the invention may be administered to an individual requiring it, at a single daily dose of 0.2 g containing the I-4573 bacterial strain of the invention in an amount equivalent to an amount of between 10⁷ and 10¹⁴ cfu, preferably 10¹¹ cfu.

As previously mentioned, daily dosages may also range from 10⁴ to 10¹⁵ total cell count (TCC), more preferably from 10⁸ to 10¹³ TCC of the *F*. *prausnitzii* strain of the invention as a medicament, for example as a daily dose equivalent to 10¹³ TCC.

A composition for use according to the invention may be administered to an individual requiring it from 1 to 10 times a day, each dose containing the I-4573 bacterial strain of the invention in an amount of for example between 5.10⁸ and 5.10¹¹ TCC, so that the total daily dose of the I-4573 bacterial strain of the invention administered to the individual is as indicated above.

In particular, a composition for use according to the invention may be administered to an individual for 1 day, 2 days, 3 days, 4 days, 5 days, 6 days, 1 week, 10 days, 14 days, 15 days, 3 weeks, 1 month or for more than 1 month. When a composition for use according to the invention is administered for more than 1 day, the same daily amount of the *F. prausnitzii* strain of the invention can be administered every day or the daily amount can vary along the treatment period. In particular, the daily dosage of the *F*. *prausnitzii* strain of the invention may be higher during the first days of the treatment and then gradually decrease.

A composition according to the invention may also comprise at least one among: antioxidants, fish oils, DHA, EPA, vitamins, minerals, phytonutrients, a protein, a lipid, probiotics, and combinations thereof.

An individual according to the invention may accordingly be:
- an individual which will start or has already started an antibiotic treatment; and/or
- an individual which is finishing or has just finished an antibiotic treatment; and/or
- an individual which will start or has already started a Proton pump inhibitors (PPI) treatment; and/or
- an individual which is finishing or has just finished a Proton pump inhibitors (PPI) treatment; and/or
- an individual who has been treated for an episode of *C*. *difficile* infection, for example a first episode of *C*. *difficile* infection, for example with vancomycine and/or fidaxomicine; and/or
- an individual with a first episode of *C*. *difficile* infection or with a recurrent *C. difficile* infection.

The *Clostridioides difficile* infection to be treated and/or prevented according to the invention may in particular occur after the use of at least one antibiotic and/or PPI medication by the considered individual. Accordingly, an individual according to the invention may in particular be an individual which will start or has already started an antibiotic and/or PPI treatment or with a dysbiosis.

A bacterial strain for use according to the invention, a culture supernatant thereof, or a composition comprising it, may accordingly be administered simultaneously and/or separately, in particular simultaneously, with at least one antibiotic medication (antibiotic) of the antibiotic treatment and/or with at least one and/or PPI medication, such as for example separately during the same day, or even separately with at least one, or even more, days between the administration of the bacterial strain and the administration of the antibiotic/PPI medication.

In a particular embodiment, the bacterial strain for use according to the invention or culture supernatant thereof and the at least one antibiotic and/or PPI are administered to the individual in the same composition.

In a particular embodiment, the bacterial strain for use according to the invention or culture supernatant thereof and the at least one antibiotic and/or PPI are administered in separate compositions.

When the bacterial strain for use according to the invention or culture supernatant thereof and the at least one antibiotic and/or PPI are administered in separate compositions, the compositions can be administered to the individual simultaneously or separately through the same route of through different routes.

By simultaneously, it is understood that the bacterial strain for use according to the invention, culture supernatant thereof, or composition comprising it, can be administered at the same moment or up to the same day or couple of days as the antibiotic and/or PPI.

By separately, it is understood that the bacterial strain for use according to the invention, culture supernatant thereof, or composition comprising it, can be administered with at least several days, for example at least three days of difference.

A bacterial strain for use according to the invention, a culture supernatant thereof, or a composition comprising it, may be administered simultaneously and separately with at least one antibiotic medication (antibiotic) of the antibiotic treatment and/or with at least one and/or PPI medication, such as for example the bacterial strain for use according to the invention, the culture supernatant thereof or the composition comprising it according to the invention may be administered simultaneously as the administration of the antibiotic/PPI medication and also after the end of the antibiotic and/or PPI treatment.

The dosage and frequency of administration of a bacterial strain for use according to the invention or culture supernatant thereof may be adapted depending on the host response.

For illustrative purposes only, the frequency of administration of a bacterial strain for use according to the invention, culture supernatant or composition comprising it, may be a daily administration for 5 to 15 consecutive days. The administration can alternatively be every 2, 3, 4, 5, 6 or 7 days for a period of up to several months.

In case of an individual which will start or has already started a treatment as mentioned above, and in particular an antibiotic and/or PPI treatment, the frequency of administration of a bacterial strain for use according to the invention, culture supernatant or composition comprising it, may be a daily administration all along the treatment of the individual, in particular all along the antibiotic and/or PPI treatment, and the administration of bacterial strain for use according to the invention, culture supernatant or composition comprising it :
- may be started one or several days before the start of the above-mentioned treatment, in particular antibiotic and/or PPI treatment; and/or
- may end one or several days after the end of the above-mentioned treatment, in particular antibiotic and/or PPI treatment.

As is known in the art, adjustments for protein degradation, systemic versus localized delivery, as well as the age, body weight, general health, sex, diet, time of administration, drug interaction and the severity of the condition may be necessary, and is easily determined with routine experimentation by those skilled in the art.

An antibiotic medication, or antibiotic, and/or PPI medication relevant according to the invention can be any medication which alters, inhibits the growth of or destroys microorganisms of the gut microbiota.

In a non-limiting way, such antibiotic can for example be selected from the group consisting of Penicillins, Tetracyclines, Cephalosporins, Quinolones, Lincomycins, Macrolides, Sulfonamides, Glycopeptides, Aminoglycosides, Carbapenems and mixtures thereof, and in particular selected from the group consisting of amoxicillin, doxycycline, cephalexin, ciprofloxacin, clindamycin, metronidazole, azithromycin, sulfamethoxazole and trimethoprim, clavulanate, ofloxacin, levofloxacin and mixtures thereof.

Antibiotic can for example be selected from the group consisting of amoxicillin, doxycycline, cephalexin, ciprofloxacin, clindamycin, metronidazole, azithromycin, sulfamethoxazole and trimethoprim, clavulanate, levofloxacin, vancomycin, fidaxomicin, metronidazole, kanamycin, gentamycin, colistin, clindamycin and mixtures thereof.

In a non-limiting way, such PPI can for example be selected from the group consisting of Omeprazole, lansoprazole, pantoprazole, rabeprazole, esomeprazole and dexlansoprazole.

The present invention also relates to the use of:
- a bacterial strain of the species *Faecalibacterium prausnitzii* deposited with the CNCM under accession number CNCM I-4573; and/or
- a culture supernatant thereof; and/or
- a composition comprising, in a physiologically acceptable medium, the bacterial strain or culture supernatant thereof,
for treating and/or preventing a *Clostridioides difficile* infection in an individual.

The present invention also relates to a method for treating and/or preventing a *Clostridioides difficile* infection in an individual, comprising administering to the said individual:
- a bacterial strain of the species *Faecalibacterium prausnitzii* deposited with the CNCM under accession number CNCM I-4573; and/or
- a culture supernatant thereof; and/or
- a composition comprising, in a physiologically acceptable medium, the bacterial strain or culture supernatant thereof.

The present invention also relates to the use of:
- a bacterial strain of the species *Faecalibacterium prausnitzii* deposited with the CNCM under accession number CNCM I-4573; and/or
- a culture supernatant thereof; and/or
- a composition comprising, in a physiologically acceptable medium, the bacterial strain or culture supernatant thereof,
for the manufacture of a medicament for treating and/or preventing a *Clostridioides difficile* infection in an individual.

The invention is described below in greater details by means of the following examples which are given solely by way of illustration.

All the references to percentages are percentages by weight unless otherwise indicated.

### EXAMPLES

The *F. prausnitzii* strain according to the invention, deposited with the CNCM under the accession number CNCM I-4573, was tested for its capacity to modulate, *in vitro* and *in vivo, C. difficile* intestinal colonization, in particular in mice presenting an induced dysbiosis.

Twenty 6-week-old conventional C57 Black 6 (C57BL/6) female mice were identified and caged with sterile food and water in isolators to protect them from the external environment. The mice are divided into 2 groups:
- 10 mice are treated with PBS- Glycerol 16% (Vehicle); and
- 10 mice treated with *F*. *prausnitzii* strain CNCM I-4573.

In order to create an intestinal dysbiosis, 6 days prior to infection (D-6) and for 3 days (D-6 to D-3), all the mice were given a cocktail of antibiotics in the drinking water consisting of Kanamycin 0.4 mg/mL, Gentamycin 0.035 mg/mL, Colistin 0.0567 mg/mL, Metronidazole 0.215 mg/mL and Vancomycin 0.045 mg/mL. On D-1, all mice were given a single 200 µL intraperitoneal (IP) dose of clindamycin 50 mg/kg.

From D-6 to D+14, for each group, mice are fed once daily with 200 µL of the strain corresponding to their group. On D0, all mice were infected by gastric gavage with 500 µL of *C*. *difficile* strain R20291 (DSM 27147) at a concentration of 2 × 10⁵ CFU/mL. For this purpose, pre-cultures were performed the day before with *C*. *difficile* strain R20291 (DSM 27147) and the following morning, a Gram stain was performed to verify the purity of the suspension and the culture was diluted with 27 mL of BHI with 3 mL of pre-culture. After approximately 7 hours of anaerobic culture, the suspension was enumerated and adjusted to 2×10⁵ bacteria/mL in 50 mL of regenerated 1 × PBS. Following infection with *C*. *difficile,* mice are then weighed daily for 8 days from infection and then at D10, D14 and D20.

In order to monitor intestinal colonisation by *C*. *difficile,* the faeces of the mice were collected at D1, D2, D3, D5, D8, D 12, D14 and D20 and resuspended in PBS to obtain a final concentration of 10 mg/mL. The suspensions are then diluted to 10⁻¹, 10⁻² and 10⁻³ and spread on BHI agar + 3% blood + *C*. *difficile* selective supplement (Biomérieux^{®}) and incubated for 48 hours at 37°C under anaerobic conditions. The suspension non-diluted is also spread in the same conditions.

### Results

All mice were given a cocktail of antibiotics to induce dysbiosis and allow better implantation of *C*. *difficile* R20291 (DSM 27147) in the gut. Mice were fed every day of the experiment (D-6 to D14) with *F. prausnitzii* CNCM I-4573 or PBS-Glycerol 16% (Vehicle). After infection with the toxigenic *C*. *difficile* strain R20291 (DSM 27147), clinical scores (loss of weight and activity, diarrhoea, mortality) were evaluated, colonisation was monitored by counting the bacteria in the faeces.

### 1) Clinical evaluation

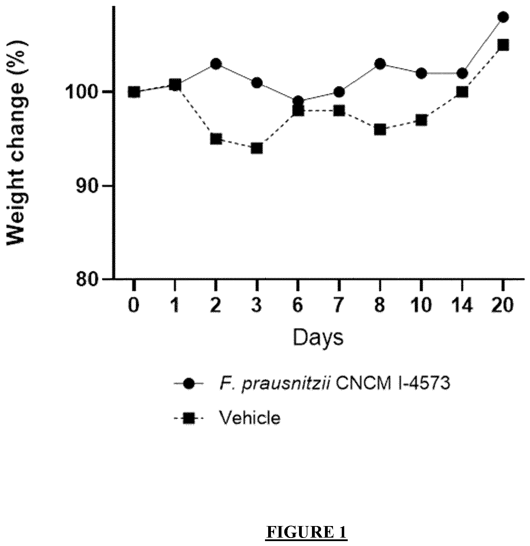

Regarding the evaluation of clinical scores (Figure 1), no weight loss, loss of activity or diarrhoea was observed in the mice treated with the *F. prausnitzii* strain of the invention.

On the other hand, for the mice in the corresponding control group (PBS - vehicle), a loss of weight from D2 onwards, then a return to their normal weight at D6 post-infection, a loss of activity and diarrhoea were observed.

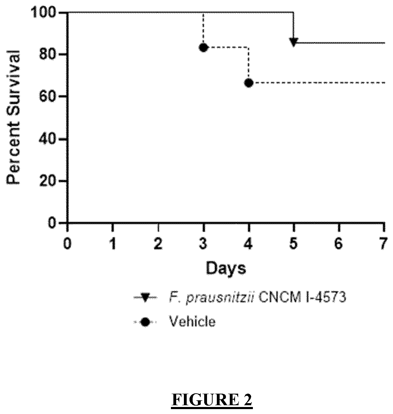

The survival of the animals after challenge was monitored. As shown in Figure 2, following infection with *C*. *difficile,* 2 mice died in the PBS-Glycerol 16% group (control - vehicle) and 1 mouse treated with *F*. *prausnitzii* CNCM I-4573 died 5 days after infection.

### 2) Monitoring of colonisation

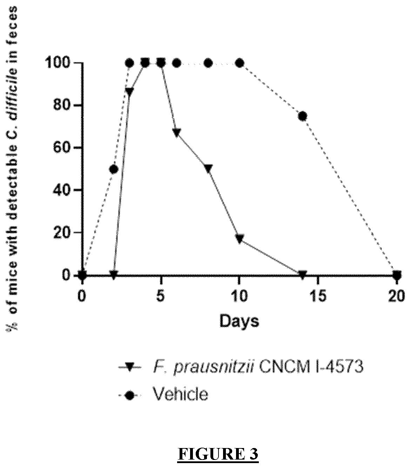

With regard to monitoring the colonisation of mice by *C*. *difficile* (Figure 3), all mice were colonised by *C*. *difficile* at D4.

The mice in the *F. prausnitzii* strain of the invention group were well colonised although they did not appear to develop symptoms. There was a delay in colonisation compared to the control group (vehicle group) and a more rapid elimination of *C*. *difficile* (from D6, compared to D14 for the control group). At D 10, the mice were still colonised by *C*. *difficile,* 4 out of 4 mice treated with PBS-Glycerol 16% (vehicle control group), 1 out of 6 mice for the group treated with the *F. prausnitzii* strain of the invention.

A treatment based on the administration of the *F. prausnitzii* strain of the invention unexpectedly demonstrated the ability to provide a protection from a *C*. *difficile* infection. The mice did not develop clinical signs (stable weight, normal faeces and activity) despite all being colonized. The colonization was delayed and a faster elimination of *C*. *difficile* was surprisingly observed.

## Claims

1. A bacterial strain of the species *Faecalibacterium prausnitzii* deposited with the CNCM under accession number CNCM I-4573, and/or a culture supernatant thereof, for use in the treatment and/or prevention of a *Clostridioides difficile* infection in an individual.

2. The bacterial strain, and/or culture supernatant thereof, for use thereof as claimed in claim 1, **characterized in that** the individual is selected from the group consisting of human beings; *Suidae*, in particular pigs, more particularly piglets; *Bovinae*, in particular cattle, more particularly bulls, and more particularly steers; *Canidae*, in particular dogs; *Equidae*, in particular horses; and *Phasianidae*, in particular poultry, more particularly chickens.

3. The bacterial strain, and/or culture supernatant thereof, for use thereof as claimed in claim 1 or 2, **characterized in that** the individual is a mammal and is more particularly a human being.

4. The bacterial strain, and/or culture supernatant thereof, for use thereof as claimed in any one of claims 1 to 3, **characterized in that** the bacterial strain of the species *Faecalibacterium prausnitzii* deposited with the CNCM under accession number CNCM I-4573 is administered to the said individual in a live, inactive and/or dead form, preferably in a live or inactive form, more particularly in a live form.

5. The bacterial strain, and/or culture supernatant thereof, for use thereof as claimed in any one of claims 1 to 4, **characterized in that** the *Clostridioides difficile* infection occurs after the use of at least one antibiotic medication and/or at least one Proton pump inhibitors (PPI) medication by the individual.

6. The bacterial strain, and/or culture supernatant thereof, for use thereof as claimed in any one of claims 1 to 5, **characterized in that** the bacterial strain of the species *Faecalibacterium prausnitzii* deposited with the CNCM under accession number CNCM I-4573, and/or the culture supernatant thereof, is administered simultaneously with and/or after the at least one antibiotic medication and/or the at least one PPI medication.

7. The bacterial strain, and/or culture supernatant thereof, for use thereof as claimed in any one of claims 1 to 6, wherein the bacterial strain of the species *Faecalibacterium prausnitzii* deposited with the CNCM under accession number CNCM I-4573 is included in a composition comprising a physiologically acceptable medium.

8. The bacterial strain, and/or culture supernatant thereof, for use thereof as claimed in claim 7, **characterized in that** the bacterial strain of the species *Faecalibacterium prausnitzii* deposited with the CNCM under accession number CNCM I-4573 is included in an oral or rectal composition, in particular in an oral composition.

9. The bacterial strain, and/or culture supernatant thereof, for use thereof as claimed in claim 7 or 8, **characterized in that** the bacterial strain is included in a pharmaceutical product.

10. The bacterial strain, and/or culture supernatant thereof, for use thereof as claimed in any one of claims 7 to 9, **characterized in that** the composition is suitable for the administration of a daily dose representing from 10⁴ to 10¹⁵ total cell count (TCC) of the bacterial strain as a medicament, preferably in the form of a daily dose representing from 10⁸ to 10¹³ TCC, more particular representing 10¹³ TCC.
